(19) Europäisches Patentamt
European Patent Office
Office européen des brevets

(11) Veröffentlichungsnummer: **0 518 110 B1**

# (12) EUROPÄISCHE PATENTSCHRIFT

(45) Veröffentlichungstag der Patentschrift: **09.08.95**

(21) Anmeldenummer: **92108773.0**

(22) Anmeldetag: **25.05.92**

(51) Int. Cl.[6]: **C07C 65/24**, A61K 31/19, C07C 51/367, C07C 51/493, C07C 51/487, C07C 69/734, C07C 67/31

## (54) Verfahren zur Herstellung der reinen Enantiomere der 4-[4-(4'-tert.Butylphenyl)-2-hydroxybutoxy]-benzoesäure und ihrer Alkylester.

(30) Priorität: **10.06.91 DE 4119055**

(43) Veröffentlichungstag der Anmeldung:
**16.12.92 Patentblatt 92/51**

(45) Bekanntmachung des Hinweises auf die Patenterteilung:
**09.08.95 Patentblatt 95/32**

(84) Benannte Vertragsstaaten:
**AT BE CH DE DK ES FR GB GR IT LI LU MC NL PT SE**

(56) Entgegenhaltungen:
**EP-A- 0 133 935**
**FR-A- 2 294 691**

(73) Patentinhaber: **Klinge Pharma GmbH**
**Berg-am-Laim-Strasse 129**
**D-81673 München (DE)**

(72) Erfinder: **Reiter, Friedemann, Dr.**
**Zugspitzstrasse 36**
**W-8011 Putzbrunn (DE)**
Erfinder: **Henschel, Hans-Helmut**
**Gudrunstrasse 18**
**W-8000 München 19 (DE)**

(74) Vertreter: **von Hellfeld, Axel, Dr. Dipl.-Phys. et al**
**Wuesthoff & Wuesthoff**
**Patent- und Rechtsanwälte**
**Schweigerstrasse 2**
**D-81541 München (DE)**

EP 0 518 110 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

In der EP 0 133 935 werden hypolipämisch wirksame p-Oxybenzoesäurederivate, Verfahren zu ihrer Herstellung und ihre Verwendung als Arzneimittel beansprucht. Von diesen hochwirksamen und gut verträglichen Wirkstoffen hat sich im therapeutischen Einsatz insbesondere die 4-[4-(4'-tert.Butylphenyl)-2-hydroxybutoxy]-benzoesäure(1) bewährt, die unter der Bezeichnung LIFIBROL [INN] bekannt geworden ist.

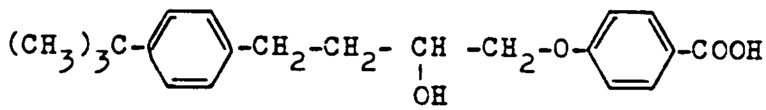 (1)

Da LIFIBROL ein Asymmetriezentrum besitzt, fällt es bei seiner Herstellung nach dem beanspruchten Verfahren in Form eines Enantiomerengemisches [(1a) + (1b)] an.

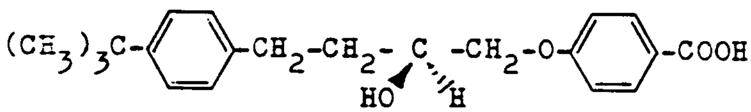 (1a) S(+)-LIFIBROL

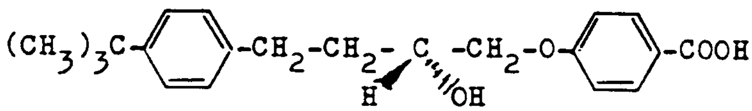 (1b) R(-)-LIFIBROL

Nach derzeitiger Auffassung über den Nachweis von Wirksamkeit und Unbedenklichkeit eines neuen chiralen Wirkstoffes ist die Zurverfügungstellung seiner reinen enantiomeren Formen unumgänglich. Bekanntlich können sich Enantiomere sowohl in der Bindung an Proteine - insbesondere Receptoren - als auch im kinetischen und metabolischen Verhalten deutlich unterscheiden und damit sehr unterschiedliche pharmakologische und toxikologische Wirkungen besitzen. Jedoch auch bei praktisch gleichen Eigenschaften ist dem reinen Enantiomer der Vorzug zu geben, da gegenüber dem Racemat die Analytik vereinfacht ist und eine bessere Kontrolle am biologischen Objekt möglich ist.
Für die Gewinnung reiner enantiomerer Formen kommen im Prinzip nur zwei Verfahren in Frage, entweder die Isolierung aus dem Racemat oder die asymmetrische Synthese.

Eine wirtschaftliche Isolierung aus dem Racemat erscheint wenig aussichtsreich, da die Asymmetrie im Zentrum des LIFIBROL-Moleküls wenig ausgeprägt ist und bei einer Derivatisierung des Enantiomerengemisches zu den entsprechenden Diastereomeren nur geringfügige physikochemische Unterschiede zu erwarten sind.Dies gilt auch für präparative Trennungen an chiralen Säulen.

Es konnte jedoch ein Syntheseverfahren gefunden werden,das die Herstellung der reinen Enantiomere des LIFIBROL und seiner Alkylester in überraschend guter Ausbeute ermöglicht.

Durch Umsetzung der reinen enantiomeren Formen von S(-)- bzw. R(+)-4-(4'-tert.Butylphenyl)-1,2-epoxybutan mit 4-Hydroxybenzoesäurealkylester bei erhöhter Temperatur in DMF und in Gegenwart von 4-Hydroxybenzoesäurealkylester Natriumsalz lassen sich die stereochemisch reinen Alkylester des LIFIBROL in hoher Ausbeute gewinnen und durch milde alkalische Verseifung und anschließendem Ansäuern in guter Ausbeute in die entsprechenden Enantiomere überführen.

Die für die Synthese notwendigen Vorstufen wie S(-)- oder R(+)-4-(4'tert.Butylphenyl)-1,2-epoxybutan werden durch Umsetzung von 4-tert.Butylbenzylmagnesiumchlorid mit im Handel erhältlichen, stereochemisch reinen Formen von Glycidyltosylat, oder Epichlorhydrin erhalten.

In folgenden Beispielen wird die Erfindung naher erläutert.

Beispiel 1

R(-)-4-[4-(4'-tert.Butylphenyl)-2-hydroxybutoxy]benzoesäuremethylester

200 g (0.978 Mol) R(+)-4-(4'-tert.Butylphenyl)-1,2-epoxybutan werden mit 149 g (0.980 Mol) 4-Hydroxybenzoesäuremethylester und 17.0 g (0.098 Mol) 4-Hydroxybenzoesäuremethylester Natriumsalz in 660 ml Dimethylformamid (DMF) 5 h unter Rühren auf 125° C erhitzt. Anschließend gießt man die erkaltete

Mischung in Wasser, extrahiert zweimal mit Ethylacetat und wäscht die vereinigten organischen Phasen mehrmals mit 1 N NaOH und Wasser.Nach dem Trocknen über $Na_2SO_4$ wird das Lösungsmittel i.Vak. abgezogen und ein brauner,zähflüssiger Rückstand erhalten.
Rohausbeute 327 g (94%);Farblose Kristalle aus Methylcyclohexan oder Methylcyclohexan/Diisopropylether

| | |
|---|---|
| Schmp.: | 62 - 64° C |
| $[\alpha]_D^{20}$ : | -9.5° [$CH_2Cl_2$ , c = 2 ] |

$^1$H-NMR-Spektrum ($CDCl_3$) :
1.31 s (9) ($C\underline{H}_3)_3C$
1.70 - 2.07 m (2) $ArCH_2C\underline{H}_2$
2.44 d (1) OH
2.60 - 2.97 m (2) $ArC\underline{H}_2CH_2$
3.70 - 4.20 und 3.87 s(6) $C\underline{H}$-$C\underline{H}_2O$ und $OC\underline{H}_3$
6.84 - 8.02 m (8) Aromat

Beispiel 2

S(+)-4-[4-(4'-tert.Butylphenyl)-2-hydroxybutoxy]-benzoesäuremethylester

196 g (0.959 Mol) S(-)-4-(4'-tert.Butylphenyl)-1,2-epoxybutan werden mit 146 g (0.960 Mol) 4-Hydroxybenzoesäuremethylester und 16.7 g (0.096 Mol) 4-Hydroxybenzoesäuremethylester Natriumsalz in 660 ml DMF 5 h unter Rühren auf 125° C erhitzt und wie in Beispiel 1 beschrieben weiter aufgearbeitet.
Rohausbeute 322.5 g (94%);Farblose Kristalle aus Methylcyclohexan oder aus Methylcyclohexan/Diisopropylether

| | |
|---|---|
| Schmp.: | 63 - 64° C |
| $[\alpha]_D^{20}$ : | + 10.75° [$CH_2Cl_2$ , c = 2 ] |
| $^1$H-NMR-Spektrum ($CDCl_3$) : | identisch mit dem R(-)-Enantiomeren |

Beispiel 3

R(-)-4-[4-(4'-tert.Butylphenyl)-2-hydroxybutoxy]-benzoesäure

R(-)-LIFIBROL

178 g (0.500 Mol),nach Beispiel 1 hergestellter roher R(-)-4-[4-(4'-tert.Butylphenyl)-2-hydroxybutoxy]-benzoesäuremethylester, werden mit einer Lösung von 66 g KOH in 425 ml Methanol und 100 ml $H_2O$ 1 h gerührt und über Nacht stehen gelassen.Anschließend wird die Mischung in 2,25 l $H_2O$ eingegossen,zur Entfernung neutraler Anteile dreimal mit tert.Butylmethylether extrahiert und mit 100 ml konz. HCl angesäuert.Das sich abscheidende Produkt wird in Ethylacetat aufgenommen und die organische Phase mit Wasser gewaschen.Nach dem Trocknen über $Na_2SO_4$ wird das Lösungsmittel i.Vak. abgezogen und der Rückstand aus Acetonitril und aus Methanol/$H_2O$ (8:2) kristallisiert.

| | |
|---|---|
| Ausbeute: | 109.5 g (64 %) |
| Schmp. : | 114 - 117° C |
| $[\alpha]_D^{20}$ : | - 12.7° [$CH_2Cl_2$ , c = 2] |
| IR-Spektrum(KBr) | $\nu$(OH)3600 bis 2400 $cm^{-1}$ |
| | $\nu$(C=C)1680 $cm^{-1}$ |

$^1$H-NMR-Spektrum ($CDCl_3$) :
1.31 s (9) ($C\underline{H}_3)_3C$
1.77 - 2.10 m (2) $ArCH_2C\underline{H}_2$
2.60 - 3.00 m (2) $ArC\underline{H}_2CH_2$
3.73 - 4.20 m (3) $C\underline{H}C\underline{H}_2O$
6.87 - 8.08 m (10) Aromat ,O$\underline{H}$ , COO$\underline{H}$

Beispiel 4

S(+)-4-[4-(4'-tert.Butylphenyl)-2-hydroxybutoxy]-benzoesäure

S(+)-LIFIBROL

178 g (0.500 Mol), nach Beispiel 2 hergestellter roher S(+)-4-[4-(4'-tert.Butylphenyl)-2-hydroxybutoxy]-benzoesäuremethylester werden wie in Beispiel 3 beschrieben verseift und aufgearbeitet.

| | |
|---|---|
| Ausbeute: | 109 g (63 %) |
| Schmp. : | 114 - 116° C |
| $[\alpha]_D^{20}$ : | + 12.4° [$CH_2Cl_2$ , c = 2 ] |
| $^1$H-NMR-Spektrum ($CDCl_3$) : | identisch mit dem des R(-)-Enantiomeren |

Herstellung der Vorstufen

Beispiel 5a

S(-)-4-(4'-tert.Butylphenyl)-1,2-epoxybutan

Die aus 16.5 g (0.679 g-Atom)Magenesiumspänen und 118.0 g (0.650 Mol) 4-tert.Butylbenzylchlorid in 650 ml trockenem Tetrahydrofuran (THF) unter Argon frisch bereitete Grignardlösung wird auf - 20° C gekühlt. Innerhalb 10 min läßt man bei - 20° bis - 30° C 120 ml Dilithiumtetrachlorocupratlösung (0.1 M in THF) zutropfen und rührt weitere 15 min,bevor auf - 55°C gekühlt wird. Hierzu tropft man bei -50 bis - 55° innerhalb 50 min eine Lösung von 91.3 g (0.400 Mol) S(+)-Glycidyltosylat in 400 ml THF und rührt nach beendeter Zugabe bei gleicher Temp. 1.75 h weiter. Anschließend wird innerhalb 30 min ein Lösungsmittelgemisch aus 30 ml Wasser und 100 ml THF zugetropft,und nach Entfernen des Kühlbades solange weiter gerührt bis die Temp. auf - 20° angestiegen ist.Daraufhin rührt man 500 ml 15proz. $NH_4Cl$-Lösung ein, trennt die org.Phase ab und extrahiert die wäßrige mit tert.Butylmethylether.Die vereinigten org. Phasen werden mit verd. $NH_4Cl$-Lösung gewaschen und über $Na_2SO_4$ getrocknet.Nach Abziehen der Lösungsmittel im Vak. bleibt ein zähflüssiger, gelber Rückstand, der in 300 ml Methanol aufgenommen und filtriert wird.Die klare Lösung wird mit 150 ml 20proz. Natriumethanolatlösung versetzt, 1.5 h bei Raumtemp. gerührt und in 1.6 l $H_2O$ gegossen.Das Produkt wird in Ethylacetat aufgenommen,mit Wasser gewaschen und über $Na_2SO_4$ getrocknet. Nach Abziehen des Lösungsmittels im Vak. wird das Rohprodukt bei 60 bis 65° C einer Kurzwegdestillation bei 0.06 mbar unterworfen. Schwach gelbliche, ölige Flüssigkeit.

Ausbeute: 77.3 g ( 94.6 %)
$[\alpha]_D^{20}$ : -12.0° ($CH_2Cl_2$ , c = 2 )
$^1$H-NMR-Spektrum ($CDCl_3$) :
1.33 s (9) $(CH_3)_3C$
1.73 - 1.95 m (2) $ArCH_2CH_2$
2.45 - 3.08 m (5) $ArCH_2CH_2$ ,

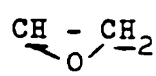

7.25(Zentrum)m (4) Aromat

Beipiel 5b

S(-)-4-(4'-tert.Butylphenyl)-1,2-epoxybutan

Die aus 12.5 g (o.514 g-Atom) Magnesiumspänen und 91.2 g (0.500 Mol) 4-tert.Butylbenzylchlorid in 500 ml trockenem THF unter Argon frisch bereitete Grignardlösung wird auf - 20°C gekühlt und mit 3.10g (0.015 Mol) Kupfer(I)-bromid -Dimethylsulfidkomplex versetzt. Man rührt 15 min bei - 25 bis - 30°C. Nach dem Abkühlen auf - 65°C tropft man innerhalb 30 min eine Lösung von 68.4 g (0.300 Mol) S(+)-Glycidyltosylat in 300 ml THF zu und rührt nach beendeter Zugabe bei gleicher Temp. noch 1.75 h weiter. Die weitere Aufarbeitung erfolgt wie in Beipiel 5a beschrieben. Das anfallende Rohprodukt ( 67.o g) wird an

einer Kieselgelsäule mit Dichlormethan/Petrolether (40-60°)[8:2] chromatographisch gereinigt.

Ausbeute: 54.0 g (88%)

$[\alpha]_D^{20}$ : - 12.8° ($CH_2Cl_2$, c = 1)

Beispiel 6a

R(+)-4-(4'-tert.Butylphenyl)-1,2-epoxybutan

Die aus 12.5 g (0.514 g-Atom)Magnesiumspänen und 91.2 g (0.500 Mol) 4-tert.Butylbenzylchlorid in 500 ml trockenem THF unter Argon frisch bereitet Grignardlösung wird auf - 20°C gekühlt und mit 100 ml Dilithiumtetrachlorocupratlösung [0.1 M in THF] versetzt, und weitere 15 min gerührt,bevor auf -55°C gekühlt wird. Hierzu tropft man innerhalb 30 min eine Lösung von 68.4 g (0.300 Mol) R(-)-Glycidyltosylat in 300 ml THF zu und rührt nach beendeter Zugabe bei gleicher Temp. noch 1.75 h weiter. Die weitere Aufarbeitung erfolgt wie in Beispiel 5a beschrieben.

Ausbeute: 51.3g (83 %)

$[\alpha]_D^{20}$ : + 12.0° ($CH_2Cl_2$, c = 1)

Nach Chromatographie an Kieselgel mit $CH_2Cl_2$

Ausbeute: 44.0g (72 %)

$[\alpha]_D^{20}$ : + 13.5° ($CH_2Cl_2$, c = 1)

$^1$H-NMR-Spektrum ($CDCl_3$): identisch mit dem des S(-)-Enantiomeren

Beispiel 6b

R(+)-4-(4'-tert.Butylphenyl)-1,2-epoxybutan

Die aus 38.9 g (1.60 g-Atom) Magnesiumspänen und 292 g (1.60 Mol) 4-tert.Butylbenzylchlorid in 1.600 ml trockenem THF unter Argon frisch bereitete Grignardlösung wird bei 5 bis 10°C innerhalb 1.5 h mit 92.5 g (1.00 Mol) R (-)Epichlorhydrin in 300 ml THF versetzt und noch weitere 3 h ohne Kühlung gerührt. Daraufhin rührt man bei 10 bis 20°C 400 ml 15proz. $NH_4Cl$-Lösung ein,trennt die org. Phase ab und extrahiert die wäßrige mit 250 ml THF.Die vereinigten org. Phasen werden mit verd. $NH_4Cl$-Lösung gewaschen und über $Na_2SO_4$ getrocknet.Nach Abziehen der Lösungsmittel im Vak. wird der Rückstand in 400 ml Methanol gelöst, die filtrierte Lösung mit 330 ml einer 20proz. Natriummethylatlösung versetzt, 1.5 h bei Raumtemp. gerührt und in 1.6 l $H_2O$ gegossen. Das Produkt wird in Ethylacetat aufgenommen,mit Wasser gewaschen und über $Na_2SO_4$ getrocknet.Nach Abziehen des Lösungsmittels im Vak. wird das Rohprodukt bei 60 - 65°C einer Kurzwegdestillation bei 0.2 mbar unterworfen

Ausbeute: 137.4 g (67%)

$[\alpha]_D^{20}$ : + 13.4° ($CH_2Cl_2$ ,c = 1)

Vorstehend wurde als Lösungsmittel Dimethylformamid angegeben. Dieses Lösungsmittel wird bevorzugt angewendet. Stattdessen sind aber auch andere Lösungsmittel einsetzbar, wie niedere Alkohole mit $C_1$-$C_4$-Ketten.

Weiterhin wurde in den vorstehenden Ausführungsbeispielen angegeben, daß die Umsetzung in Gegenwart einer geringen Menge Alkali erfolgt. Das Alkali wird in Form eines Natriumsalzes des betreffenden Hydroxybenzoesäurealkaliesters eingebracht. Statt einer solchen Einbringung eines Natriumsalzes kann auch ein Alkali in Form von Natriumhydroxyd oder Kaliumhydroxyd eingebracht werden.

**Patentansprüche**

**1.** Verfahren zur Herstellung der reinen Enantiomere des LIFIBROL und seiner Alkylester dadurch gekennzeichnet, daß man die jeweilige enantiomere Form von 4-(4'-tert.Butylphenyl)-1,2-epoxybutan mit 4-Hydroxybenzoesäurealkylestern der allgemeinen Formel (1)

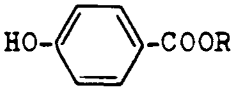

(1)

wobei R einen kurzen Alkylrest darstellt, in Gegenwart der Natriumsalzform des gleichen 4-Hydroxybenzoesäurealkylesters bei erhöhter Temperatur umsetzt, anschließend das rohe Umsetzungsprodukt

abtrennt und entweder durch Umkristallisation den stereochemisch reinen LIFIBROL-Ester gewinnt, oder durch milde alkalische Verseifung und nachfolgendem Ansäuern das ausgefällte LIFIBROL-Enantiomere in reiner,kristalliner Form herstellt.

**2.** Verfahren nach Anspruch 1 dadurch gekennzeichnet daß als enantiomere Ausgangsprodukte S(-)-4-(4'-tert.Butylphenyl)-1,2-epoxybutan und R(+)-4-(4'-tert.Butylphenyl)-1,2-epoxybutan eingesetzt werden.

**3.** Verfahren nach Anspruch 1 dadurch gekennzeichnet, daß zur Umsetzung der Methyl-,Ethyl- oder der Propylester der 4-Hydroxybenzoesäure verwendet wird.

**4.** Verfahren nach Anspruch 1 dadurch gekennzeichnet, daß die Umsetzung in Dimethylformamid bei Temperaturen zwischen 110 und 140° erfolgt.

**5.** Verfahren nach Anspruch 1 dadurch gekennzeichnet daß die Verseifung der LIFIBROL-Ester zwischen 10 und 30° erfolgt.

## Claims

**1.** Process for preparing the pure enantiomers of LIFIBROL and its alkyl esters, characterized in that the respective enantiomeric form of 4-(4'-tert.butylphenyl)-1,2-epoxybutane is reacted with 4-hydroxybenzoic acid alkyl esters of the general formula (1)

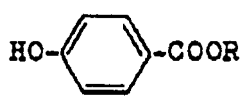

(1)

wherein R is a short alkyl radical, in the presence of the sodium salt form of the same 4-hydroxybenzoic acid alkyl ester at elevated temperature, thereafter the raw reaction product separated and either the stereo-chemically pure LIFIBROL ester is recovered by recrystallization or the precipitated LIFIBROL enantiomer is prepared in pure crystalline form by mild alkaline saponification and subsequent acidification.

**2.** Process according to claim 1, characterized in that as an enantiomeric starting product S(-)-4-(4'-tert.butylphenyl)-1,2-epoxy butane and R(+)-4-(4'-tert.butylphenyl)-1,2-epoxy butane are employed.

**3.** Process according to claim 1, characterized in that for the reaction the methyl, ethyl or propyl ester of the 4-hydroxy benzoic acid is employed.

**4.** Process according to claim 1, characterized in that the reaction is carried out in dimethylformamide at temperatures between 110 and 140°.

**5.** Process according to claim 1, characterized in that the saponification of the LIFIBROL ester is carried out between 10 and 30°.

## Revendications

**1.** Procédé de préparation des énantiomères purs du LIFIBROL et de ses esters alkyliques, caractérisé en ce qu'on fait réagir la forme énantiomère correspondante du 4-(4'-t-butylphényl)-1,2-époxybutane avec des esters alkyliques d'acide 4-hydroxybenzoïque de formule générale (1) :

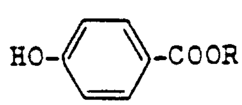

(1)

dans laquelle R représente un résidu alkyle inférieur, en présence de la forme de sel de sodium du même ester alkylique d'acide 4-hydroxybenzoïque à une température élevée, en ce qu'ensuite on

sépare le produit réactionnel brut et que soit on obtient par recristallisation l'ester de LIFIBROL stéréochimiquement pur, soit on prépare par saponification alcaline douce et par acidification ultérieure l'énantiomère de LIFIBROL précipité sous sa forme cristalline pure.

**2.** Procédé selon la revendication 1, caractérisé en ce qu'on utilise, en tant que produits de départ énantiomères, le S(-)-4-(4'-t-butylphényl)-1,2-époxybutane et le R(+)-4-(4'-t-butylphényl)-1,2-époxybutane.

**3.** Procédé selon la revendication 1, caractérisé en ce qu'on utilise pour la réaction l'ester méthylique, éthylique ou propylique de l'acide 4-hydroxy-benzoïque.

**4.** Procédé selon la revendication 1, caractérisé en ce que la réaction a lieu dans du diméthylformamide à des températures comprises entre 110 et 140 °C.

**5.** Procédé selon la revendication 1, caractérisé en ce que la saponification des esters de LIFIBROL a lieu entre 10 et 30 °C.